# EUROPEAN PATENT SPECIFICATION

(11) **EP 3 117 421 B1**
(45) Date of publication and mention of the grant of the patent: **19.02.2020**
(21) Application number: 15714707.5
(22) Date of filing: 13.03.2015
(51) Int. Cl.: G09B 23/28

(54) **ADVANCED FIRST ENTRY MODEL FOR SURGICAL SIMULATION**
ERWEITERTES ERSTEINTRAGSMODELL FÜR CHIRURGISCHE SIMULATION
MODÈLE AVANCÉ DE PREMIÈRE ENTRÉE POUR LA SIMULATION CHIRURGICALE

(30) Priority: 13.03.2014 US 201461952289 P
(43) Date of publication of application: 18.01.2017
(60) Divisional application: 19215545.5
(73) Proprietor: Applied Medical Resources Corporation, Rancho Santa Margarita, CA 92688 (US)
(72) Inventor: WACHLI, Serene, Rancho Santa Margarita, CA 92688 (US); HOFSTETTER, Gregory, K., Rancho Santa Margarita, CA 92688 (US); BLACK, Katie, Rancho Santa Margarita, CA 92688 (US); POULSEN, Nikolai, Rancho Santa Margarita, CA 92688 (US); HOLMES, Heidi, Rancho Santa Margarita, CA 92688 (US); FELSINGER, Natasha, Rancho Santa Margarita, CA 92688 (US); BRESLIN, Tracy, Rancho Santa Margarita, CA 92688 (US); PRAVONGVIENGKHAM, Kennii, Rancho Santa Margarita, CA 92688 (US); PRAVONG, Boun, Rancho Santa Margarita, CA 92688 (US); BOLANOS, Eduardo, Rancho Santa Margarita, CA 92688 (US); FALKENSTEIN, Zoran, Rancho Santa Margarita, CA 92688 (US); HART, Charles, C., Rancho Santa Margarita, CA 92688 (US); TALWAR, Tina, Rancho Santa Margarita, CA 92688 (US)
(74) Representative: Dolleymores
(86) International application number: PCT/US2015/020574
(87) International publication number: WO 2015/138982

(56) References cited:
- WO-A1-94/06109
- WO-A1-96/42076
- US-A1- 2005 214 727
- US-A1- 2012 082 970
- US-A1- 2013 157 240

## Description

### Field of the Invention

This application relates to surgical training tools, and in particular, to simulated tissue structures and models for teaching and practicing surgical procedures.

### Background of the Invention

Laparoscopic surgery requires several small incisions in the abdomen for the insertion of trocars or small cylindrical tubes approximately 5 to 10 millimeters in diameter through which surgical instruments and a laparoscope are placed into the abdominal cavity. The laparoscope illuminates the surgical field and sends a magnified image from inside the body to a video monitor giving the surgeon a close-up view of organs and tissues. The surgeon watches the live video feed and performs the operation by manipulating the surgical instruments placed through the trocars.

The first step in laparoscopic surgery is to make a small incision to access the abdomen and create pneumoperitoneum. Pneumoperitoneum is the insufflation of the abdominal cavity with carbon dioxide gas. Insufflation with gas creates a working space in the abdomen necessary for laparoscopy. Once a proper working space has been created, surgical instruments can be inserted for performing a laparoscopic procedure. This process of penetrating the abdomen and creating pneumoperitoneum prior to insertion of other instruments is called first entry. There are many different ways to achieve pneumoperitoneum. One option is using a Veress needle. A Veress needle is approximately 12-15 centimeters long with a diameter of approximately 2 millimeters. The surgeon inserts the spring-loaded needle into the abdomen of the patient after making a small incision. When the needle breaches the inner abdominal space, the spring-loaded inner stylet springs forward to cover the sharp needle in order protect internal organs. The surgeon relies on the feel and sound of the needle and spring for proper placement. Once proper entry is confirmed, carbon dioxide is introduced through the Veress needle and into the abdominal cavity of the patient expanding the abdomen to creating a working space.

Another option is a Hasson technique or cut down technique in which the surgeon makes an initial incision at the umbilicus and the tissue is bluntly dissected. A suture is placed on either side of the incision into the fascia layer to help hold the device in place. The supraperitoneal tissue is dissected away and the peritoneum is incised to enter the abdominal cavity. At this point, a Hasson trocar is inserted into the incision. The Hasson trocar has a blunt tip with suture ties and/or a balloon to hold it in place. After the trocar is placed into the incision, the device is secured with sutures and/or the balloon and carbon dioxide gas is pumped into the patient through the trocar to achieve pneumoperitoneum.

Another option is direct trocar entry. In this option, the surgeon uses a bladed or non-bladed trocar. The trocar can be used optically in which a specialized trocar is configured to receive a laparoscope and a laparoscope is inserted into the trocar before entry in order to view the penetration as it occurs. Also, the trocar may be use non-optically without a laparoscope inside. After the initial incision is made, the trocar is placed through the layers of the abdomen. Since the camera is present, all of the layers of the abdominal wall can be observed during penetration. Once the surgeon sees that he or she has broken through the peritoneum, penetration can halt, the obturator tip of the trocar pulled back slightly or removed entirely and insufflation can commence by pumping carbon dioxide gas in through the cannula to create pneumoperitoneum.

Another option involves a specialized first entry trocar such as the FIOS ® first entry trocar made by Applied Medical Resources Corporation in California. Like optical direct trocar entry, a laparoscope is inserted into the FIOS ® trocar and the abdominal wall layers are observed during insertion into the abdominal cavity. The specialized FIOS ® trocar has a small vent hole in the tip such that instead of requiring that the obturator of the trocar be pulled back or removed completely to introduce carbon dioxide through the cannula, carbon dioxide gas is introduced through the small vent hole in the tip of the obturator with the camera in place. Because carbon dioxide can be introduced through the tip, the FIOS ® trocar does not have to penetrate as deeply into the abdominal cavity as a traditional trocar, thereby, affording internal organs greater protection before insufflation can commence. Also, because the obturator does not have to be pulled back or removed, observation via the inserted camera can take place at the point of insufflation.

In addition to the above options for entering the abdominal cavity, generally, there are two common places on the abdomen that a surgeon must know how to enter. The most widely used location for first entry is the umbilicus. The umbilicus is a natural weakening in the abdomen where the umbilical cord was attached in the womb. In this part of the abdomen, there are no rectus muscles, arteries or veins so it is generally easier to reach the abdominal cavity. Additionally, the umbilicus is typically an easy place to hide a scar. When surgeons use the umbilicus as an entry site, particularly for the Hasson technique, clamps are often used to grab the base of the umbilicus and the umbilicus is inverted. At this point, an incision is made and the surgeon cuts down as desired and inserts the trocar or Veress needle. With optical entry, the surgeon is able to see all the layers of the abdominal wall. In this location of penetration, they are able to see the fatty tissue, linea alba, transversalis fascia and, finally, the peritoneum. Additionally, when entering at the umbilicus, the umbilical stalk should also be visible. The stalk is what remains of the umbilical cord and it stretches from the skin making up the umbilicus to the peritoneal layer.

If a patient has had a previous surgery and adhesions are suspected or a hernia is present at the site of the umbilicus, first entry may need to occur at another location. In this case, the surgeon will often enter from the left upper quadrant since there is less chance of damaging a vital organ in this location. The left upper quadrant is different from the umbilicus region in that there are muscle layers. The rectus abdominus muscles run parallel with the patient's abdomen and are found on either side of the patient's midline. Underneath the rectus abdominus muscles run the inferior epigastric veins and arteries which the surgeon must be careful to avoid. When a surgeon is entering the upper quadrant of the abdominal cavity optically, he or she is able to see the skin, fatty tissue, anterior rectus sheath, rectus abdominus, the epigastric vein, which runs through the posterior rectus sheath, and finally, the peritoneum. If the left upper quadrant is not an ideal position for a port, the surgeon may choose to enter at another location such as sub-xiphoid where subcutaneous fat, rectus sheath and peritoneum are present.

Since there are many options for first entry, it is important that surgeons have a way to learn and practice the various techniques. There is a need for an anatomical model of the umbilical region and surrounding abdomen that is anatomically correct and includes all the layers of the abdominal wall as well as the veins and arteries that run through the wall. Not only does the model have to be anatomically correct, but also, the model must provide a realistic aural and tactile sensation. For example, when using a Veress needle, two pops are generally felt as the surgeon pushes the needle through the abdominal wall. For optical entry, the surgeon needs to view all of the appropriate tissue layers in the abdominal wall. For entry through the umbilicus, the surgeon must be able to grasp and invert the umbilicus. Also, the model may be able to be used with all four first entry techniques and at multiple (umbilical and upper left quadrant at minimum) entry sites.

US patent application, publication number US 2005/0214727 A1 discloses a training or evaluation device that is aimed at providing such an anatomical model.

### Summary of the Invention

According to one aspect of the invention, a surgical training device is provided, as recited in Claim 1, and according to a second aspect of the invention, there is provided a method for simulating surgical insufflation, as recited in Claim 8.

### Brief Description of the Drawings

FIG. 1 is a top perspective view of a first entry model for use with a surgical training device according to the present invention.
FIG. 2 is top perspective view of a first entry model for use with a surgical training device according to the present invention.
FIG. 3 is a top perspective view of a laparoscopic trainer for use with a first entry model for use with a surgical training device according to the present invention.
FIG. 4 is a side, exploded view of an anatomical portion of a first entry model for use with a surgical training device according to the present invention.
FIG. 5 is a side view of an anatomical portion of a first entry model for use with a surgical training device according to the present invention.
FIG. 6 is a top planar view that is representative of more than one layer in an anatomical portion of a first entry model for use with a surgical training device according to the present invention.
FIG. 7 is a top planar view that is representative of more than one layer in an anatomical portion of a first entry model for use with a surgical training device according to the present invention.
FIG. 8 is top perspective, exploded view of a mold for a skin layer of a first entry model for use with a surgical training device according to the present invention.
FIG. 9 is a side, cross-sectional view of a mold for a skin layer for a first entry model for use with a surgical training device according to the present invention.
FIG. 10 is a top perspective view of a mold for a skin layer for a first entry model for use with a surgical training device according to the present invention.
FIG. 11 is a cross-sectional, side view of a surgical training device according to the present invention.
FIG. 12 is a cross-sectional, side view of a surgical training device according to the present invention.

### Detailed Description of the Invention

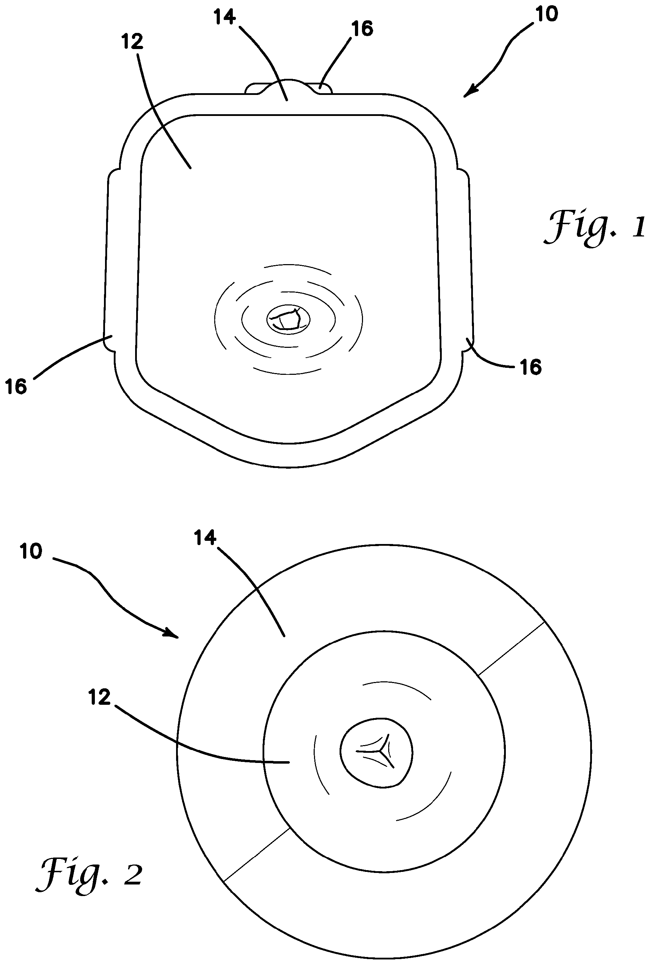

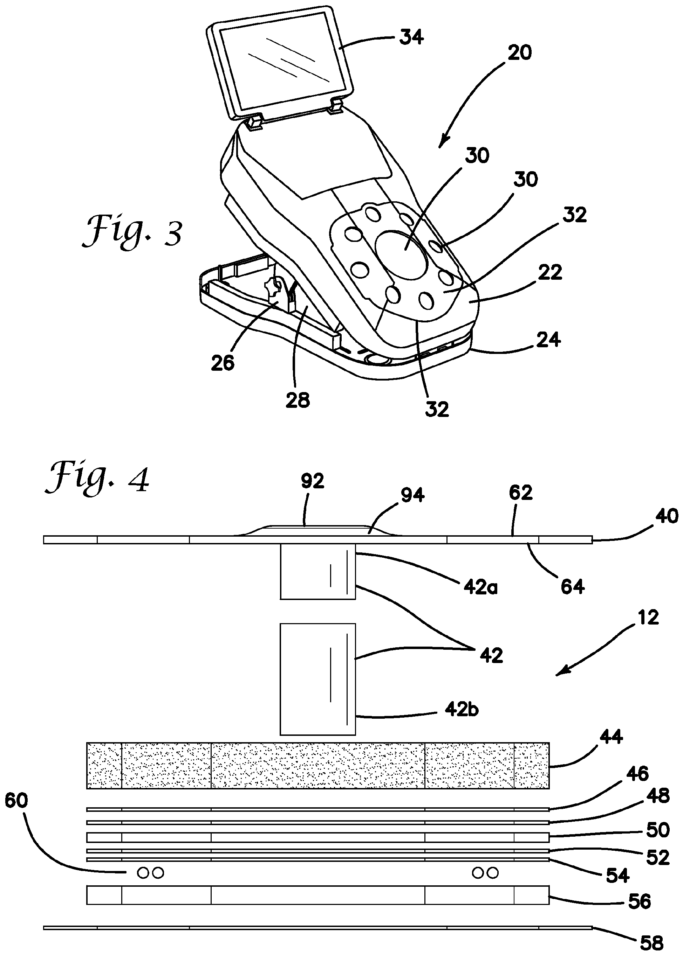

Turning now to FIG. 1, there is shown a model 10 of an abdominal region that includes the umbilicus for practicing surgical first entry into the abdominal cavity for performing laparoscopic surgical procedures. Throughout this specification the model 10 will be referred to as the first entry model 10. The model 10 includes an anatomical portion 12 connected to a support 14 to form a substantially planar configuration. The support 14 is a frame that encompasses and connects to the perimeter of the anatomical portion 12 and holds the anatomical portion 12 together. In particular, the support 14 includes a top frame and a bottom frame made of plastic material sufficiently rigid to provide structural support and maintain the planar shape of the model 10 and permit the center-located anatomical portion to be penetrated from one side to the other. In one variation, the model 10 is slightly curved to mimic an outwardly curved abdomen. The top frame and the bottom frame snap together capturing the perimeter of the anatomical portion 12 between the top and bottom frames. The model 10 in FIG. 1 is polygonal having five sides forming a slightly elongated shape wherein one side is curved outwardly in a generally U-shaped configuration. A model 10 having a circular support 14 that frames a circular anatomical portion 12 is shown in FIG. 2. The model 10 can be any shape. The frame 14 includes connecting elements 16 configured for connecting the model 10 to a larger laparoscopic trainer as shown in FIG. 3.

Turning now to FIG. 3, a laparoscopic trainer 20 includes a top cover 22 connected to a base 24 by a pair of legs 26 spacing the top cover 22 from the base 24. The laparoscopic trainer 20 is configured to mimic the torso of a patient such as the abdominal region. The top cover 22 is representative of the anterior surface of the patient and a space 28 defined between the top cover 22 and the base 24 is representative of an interior of the patient or body cavity where organs reside. The laparoscopic trainer 20 is a useful tool for teaching, practicing and demonstrating various surgical procedures and their related instruments in simulation of a patient. When assembled, the top cover 22 is positioned directly above the base 24 with the legs 26 located substantially at the periphery and interconnected between the top cover 22 and base 24 The top cover 22 and base 24 are substantially the same shape and size and have substantially the same peripheral outline. The laparoscopic trainer 20 includes a top cover 22 that angulates with respect to the base 24. The legs 26 are configured to permit the angle of the top cover 22 with respect to the base 24 to be adjusted. FIG. 3 illustrates the trainer 20 adjusted to an angulation of approximately 30-45 degrees with respect to the base 24.

For practicing various surgical techniques, surgical instruments are inserted into the cavity 28 of the laparoscopic trainer 20 through pre-established apertures 30 in the top cover 22. These pre-established apertures 30 may include seals that simulate trocars or may include simulated tissue that simulates the patient's skin and abdominal wall portions. For example, the circular first entry model 10 depicted in FIG. 2 is connected to the top cover 22 in the location of the central circular aperture 30 that has a conforming circular shape. The top cover 22 of the laparoscopic trainer 20 is configured with a removable insert 32 that is replaceable with the first entry model 10 depicted in FIG. 1. The insert 32 which is provided with apertures 30 has a shape that conforms to an opening in the top cover 22. When the insert 32 is removed, the first entry model 10, such as the one depicted in FIG. 1, having a conforming shape is inserted into the opening in the top cover 20 and the connecting elements 16 on the first entry model 10 aid in securing the model 10 to the trainer 20.

Various tools and techniques may be used to penetrate the top cover 20 as described in the background of this description to perform mock procedures not only on the model 10 but also on additional model organs placed between the top cover 22 and the base 24. When placed inside the cavity 28 of the trainer 20, an organ model is generally obscured from the perspective of the user who can then practice performing surgical techniques laparoscopically by viewing the surgical site indirectly via a video feed displayed on a video monitor 34. The video display monitor 34 is hinged to the top cover 22 and is shown in an open orientation in FIG. 3. The video monitor 34 is connectable to a variety of visual systems for delivering an image to the monitor 34. For example, a laparoscope inserted through one of the pre-established apertures 30 or a webcam located in the cavity 28 and used to observe the simulated procedure can be connected to the video monitor 34 and/or a mobile computing device to provide an image to the user. After first entry procedures are practiced on a first entry model 10 connected to the trainer 20, the first entry model 10 is removed and may be replaced with a new insert or reconstructed and reconnected to the trainer 20 to allow training to continue or be repeated. Of course, the first entry model 10 may be employed independently of the trainer 20 for practicing first entry techniques.

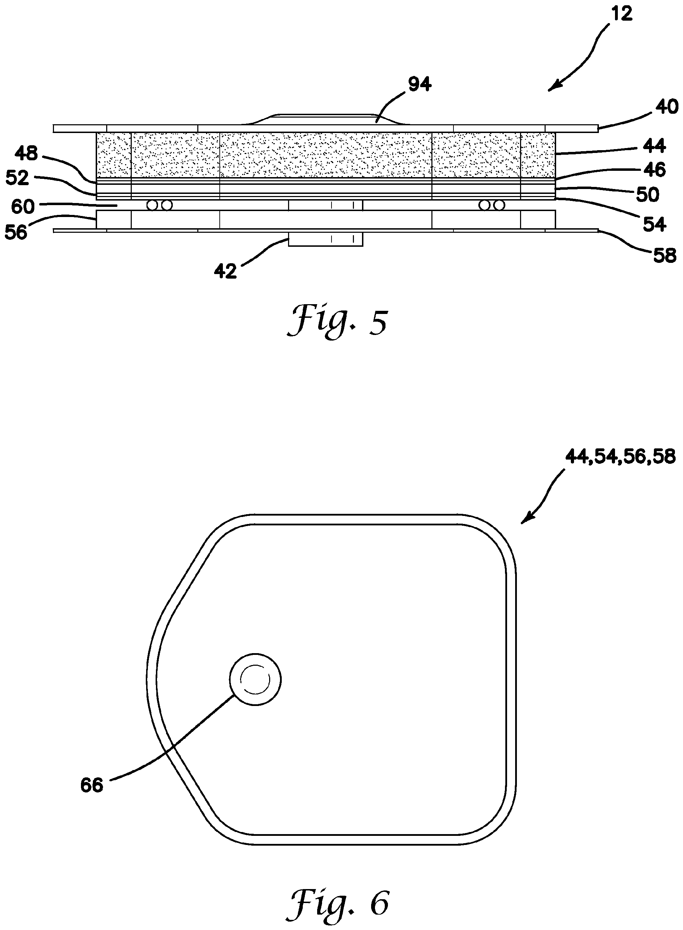

Turning now to FIGs. 4 and 5, the anatomical portion 12 of the first entry model 10 will now be described. The anatomical portion 12 includes a skin layer 40, an umbilical stalk 42, a fat layer 44, an anterior rectus sheath layer 46, a first rectus muscle layer 48, a second rectus muscle layer 50, a third rectus muscle layer 52, a posterior rectus sheath layer 54, a transversalis fascia layer 56, and a peritoneum layer 58. The layers 40, 44, 46, 48, 50, 52, 54, 56, 58 are placed one on top of the other as shown in FIGs. 5-6 with the umbilical stalk 42 penetrating through all of the layers beneath the skin layer 40. The layers 40, 44, 46, 48, 50, 52, 54, 56, 58 are connected together with adhesive or other fastener. In one variation, the layers 40, 44, 46, 48, 50, 52, 54, 56 are connected with at least one price-tag holder punched through the layers and sandwiched between the skin layer 40 and the peritoneum layer 58 before being attached to the frame 14. In another variation, the layers are held together without adhesive or other fastener and clamped between the top frame and bottom frame. An optional inferior epigastric vein and artery layer 60 is included between the posterior rectus sheath layer 54 and the transversalis fascia layer 56 as shown in FIGs. 4-5.

With continued reference to FIG. 4, the skin layer 40 is molded of silicone or thermoplastic elastomer dyed with a flesh color. The skin layer 40 includes a top surface 62 and bottom surface 64 defining a thickness of approximately 2.5mm (0.1 inches). The skin layer 40 includes an integrally formed umbilical stalk portion 42a. The skin layer 40 will be described in greater detail below.

Still referencing FIG. 4, the fat layer 44 is made of cellular polyethylene foam having a yellow color. The cellular foam layer is not solid but textured with air bubbles. The fat layer 44 is approximately 16mm (0.625 inches) thick. The anterior rectus sheath layer 46 is made of solid ethylene vinyl acetate (EVA) foam having a white color and is approximately 1 millimeter thick. The first rectus muscle layer 48 is made of solid EVA foam and is red in color and approximately 1 millimeter thick. The second rectus muscle layer 50 is made of cellular polyethylene foam having a pink color. The second rectus muscle layer 50 is cellular foam that includes air bubbles that provide a cellular texture and is approximately 3.2mm (0.125 inches) thick. The third rectus muscle layer 52 is made of solid EVA foam having a red color and is approximately 1 millimeter thick. The posterior rectus sheath layer 54 is made of solid EVA foam that is white in color and is approximately 1 millimeter thick. The transversalis fascia layer 56 is made of cellular polyethylene foam that is white in color and approximately 6.4mm (0.25 inches) thick. The fascia layer 56 has a cellular texture arising from the cellular polyethylene foam as opposed to the solid EVA foam layers. The peritoneum layer 58 is made of solid EVA foam that is white in color and approximately 1 millimeter thick. The inferior epigastric vein and artery layer 60 include solid or hollow elongate cylindrical structures made of silicone or Kraton polymer or other elastomer having a cross-sectional diameter of approximately 3.8mm (0.15 inches). The arteries are red in color and the veins are blue in color. The layers as described above provide an optical entry with a very realistic appearance to the end user.

Turning now to FIG. 6, there is shown a top planar view that is representative of the fat layer 44, the posterior rectus sheath layer 54, the transversalis fascia layer 56 and the peritoneum layer 58. These layers are approximately 152mm (6 inches) wide and 165mm (6.5 inches) long. The fat layer 44, the posterior rectus sheath layer 54, the transversalis fascia layer 56 and the peritoneum layer 58 all have a circular aperture 66 that is approximately 25mm (1 inch) in diameter. The aperture 66 is located approximately 51mm (2 inches) from one side and is in the same place in all of these layers 44, 54, 56, 58 such that when overlaid the apertures 66 line up to provide a pathway for the umbilical stalk 42 across these layers.

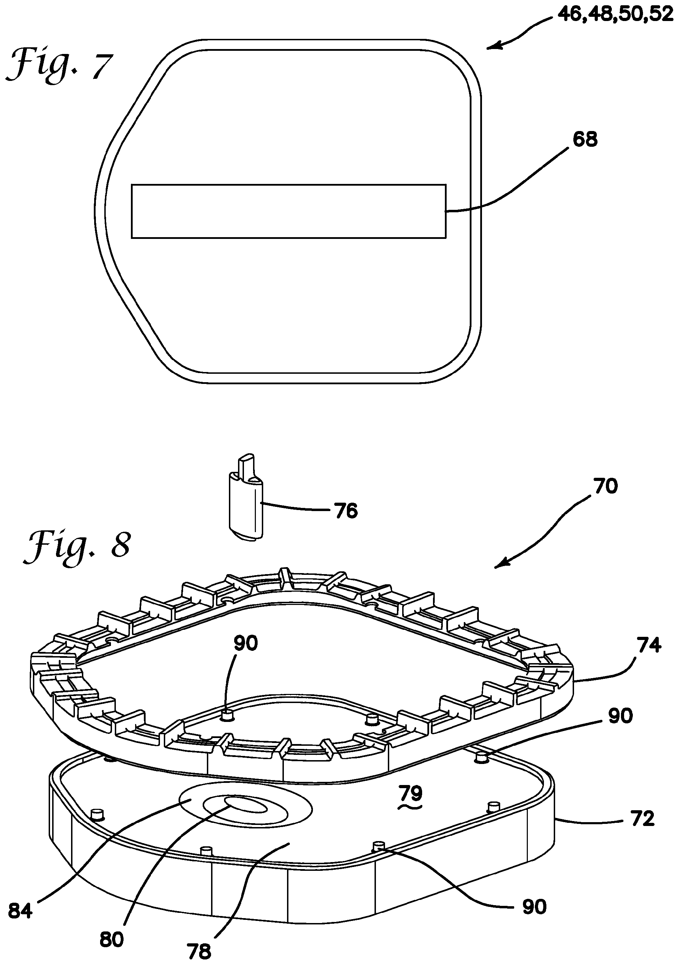

Turning now to FIG. 7, there is shown a top planar view that is representative of the anterior rectus sheath layer 46, first rectus muscle layer 48, the second rectus muscle layer 50 and the third rectus muscle layer 52. These layers are approximately 152mm (6 inches) wide and 165mm (6.5 inches) long. The anterior rectus sheath layer 46, first rectus muscle layer 48, the second rectus muscle layer 50 and the third rectus muscle layer 52 all have an elongate opening 68. The elongate opening 68 extends along the center line of the layers and is shown in FIG. 7 to be a rectangular cut out that is approximately 25mm (1 inch) wide and 146mm (5.75 inches) long. When the layers 46, 48, 50, 52 are overlaid, one on top of the other, all of the respective openings 68 are aligned. When the layers 46, 48, 50, 52 are overlaid with the other layers 44, 54, 56, 58, the apertures 66 are in communication or alignment with the elongate openings 68. The elongate opening 68 represents the linea alba of the abdomen.

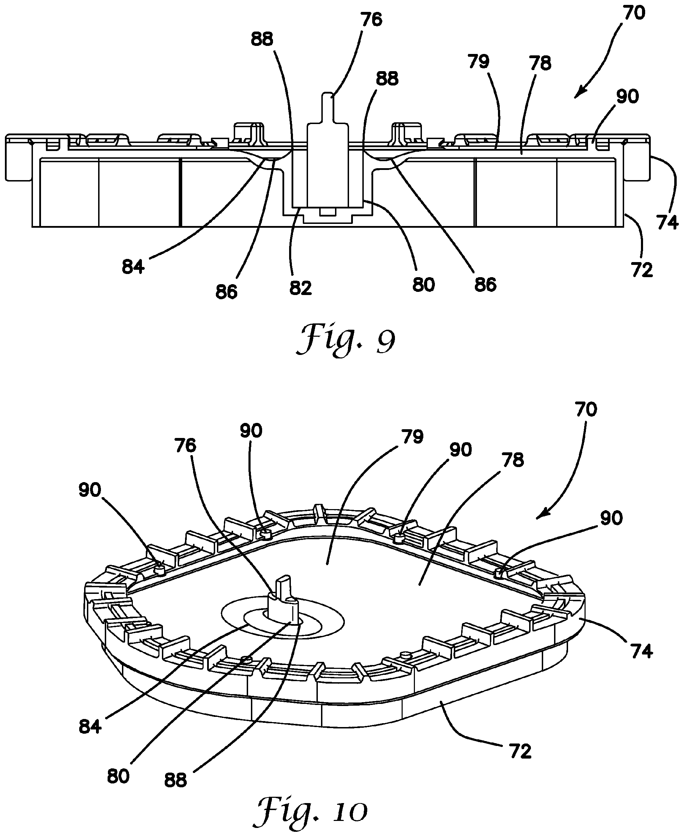

With reference back to FIG. 4 and additional reference to FIGs. 8-10, the skin layer 40 is formed by pouring the uncured and dyed silicone or thermoplastic elastomer into a special mold 70. An exploded, top perspective view of the mold 70 is shown in FIG. 8. The mold 70 includes a base 72, a top 74, and a core 76. The base 72 of the mold 70 includes a cavity 78 for receiving the plastic material. The cavity 78 is polygonal and substantially rectangular in shape. The cavity 78 includes a first floor 79 that surrounds a well 80 having a second floor 82. The second floor 82 of the well 80 is approximately 25mm (1 inch) below the first floor 79 and includes a hole for inserting the core 76 inside the well 80. The cross-section of the well 80 is elliptical in shape having a long axis of approximately 25mm (1 inch) and a short axis of approximately 13mm (0.5 inches). The cross-section of the core 76 is also elliptical in shape, complementary to the well 80. The core 76 has a long axis of approximately 19mm (0.75 inches) and a short axis of approximately 6.4mm (0.25 inches). With the core 76 in place inside the well 80 a space of approximately 3.2mm (0.125 inches) is formed all around the core 76 between the outer surface of the core 76 and the inner surface of the well 80 into which silicone or thermoplastic elastomer is poured to form a tubular structure of the umbilical stalk 42a having an opening 92. The core 76 is approximately 25mm (1 inch) and a half in length and extends above the pour line when inside the well 80.

The mold cavity 78 further includes a circumferential well 84 that is formed circumferentially around the first well 80. The circumferential well 84 has a concave or curved floor 86 that is approximately 3.2mm (0.125 inch) deeper from the first floor 79. When silicone or thermoplastic elastomer is poured, an elliptical toroidal shape with a flat top is formed in the plastic material resulting in an increased thickness of material of approximately 6.4mm (0.25 inch) in the area of the circumferential well 84 in the final product. The circumferential well 84 has an inner perimeter 88 that coincides with the wall of the first well 80. The annular distance from the inner perimeter 88 of the circumferential well 84 to the outer perimeter or end of circumferential well 84 is approximately 19mm (0.75 inches). The base 72 of the mold 70 further includes a plurality of pegs 90 upstanding from the first floor 79 to form holes in the resulting molded material. Although the first well 80 is described to have an elliptical shape, in another variation it is circular in shape with a corresponding circular core and circular circumferential well.

The core 76 is first inserted into the well 80 and silicone or thermoplastic elastomer is poured into the base 72 of the mold 70. The silicone or thermoplastic elastomer will run into the well 80 forming a tubular structure defined by the space between the core 76 and wall of the well 80. The silicone or thermoplastic elastomer will also run into the circumferential well 84 and cover the concave floor 86 forming a substantially toroidal shape of increased thickness of approximately 6.4mm (0.25 inch). The circumferential portion of increased thickness 94 is visible in FIGs. 4 and 5. The silicone or thermoplastic elastomer in its liquid state will cover the first floor 79 forming a planar area having a thickness of approximately 3.2mm (0.125 inches). The top 74 of the mold 70 will be placed over the base 72 of the mold 70. The top 74 is configured to cover only the perimeter of the poured silicone or thermoplastic elastomer to reduce the thickness of the silicone around the perimeter.

After the silicone or thermoplastic elastomer has solidified, the top 74 of the mold is removed and the molded silicone or thermoplastic elastomer is removed from the mold 70. The core 76 is also removed from the material leaving an elliptical opening 92 through the skin layer 40. The tubular structure or umbilical stalk 42a that is integrally formed by the well 80 with the rest of the skin layer 40 defines an opening 92 and is elliptical in shape having long axis of approximately 19mm (0.75 inches) and a short axis of approximately 6.4mm (0.25 inches) with a wall thickness of approximately 3.2mm (0.125 inches). The tubular structure 42a is inverted, that is, it is pushed through the opening 92 such that the surface in contact with the floor 79 of the mold 70 becomes the skin layer top surface 62. This advantageously permits the floor 79 of the mold to include texturing that would impart skin-like texture to the skin layer top surface 62. Also, by inverting the tubular structure 42a, not only an umbilical stalk is formed, but also, the portion of increased thickness 94 of the skin layer 40 will advantageously create a raised surface at the skin layer top surface 62 which is clearly visible in FIGs. 4 and 5. This raised portion 94 advantageously provides extra thickness of material for drawing sutures through and maintaining them in position without pulling through the silicone or thermoplastic material. Also, a circumferential raised portion 94 that surrounds the opening 92 creates a realistic belly-button effect that can be seen in FIG. 1. A variation of the skin layer 40 without the raised circumferential portion 94 is shown in FIG. 2. Although the umbilical stalk is approximately 25mm (1 inch) long, it may be molded to be longer, approximately 38mm (1.25 inches) to approximately 51 mm (2.0 inches) long. The skin layer 40 is planar sheet of molded material having a top surface 62 and a bottom surface 64 defining a skin layer thickness of approximately 2.5mm (0.1 inches). The skin layer 40 further includes an opening 92 with a tubular extension 42 integrally formed at opening 92 and interconnected with the rest of the layer 40. Surrounding the opening 92 is a circumferential raised portion 94 of increased thickness of approximately 5.1mm (0.2 inches). The raised portion 94 provides a convex outer surface that transitions into the remainder of the top surface 62 of the skin layer 40.

The mold 70 is 3D printed from Vero White Plus Fullcure 835 material. The distance from the pour line to the floor 79 is approximately 2.5mm (0.1 inches) to create a skin layer thickness of approximately 2.5mm (0.1 inches). Around the perimeter, the thickness beneath the top 74 of the mold 70 is reduced to approximately 1.3mm (0.05 inches) for a resulting skin layer thickness at the perimeter having a reduced thickness of approximately 1.3mm (0.05 inches) which facilitates connection to the frame support 14. At the circumferential well 84 location, the thickness of the resulting skin layer 40 is approximately 5mm (0.2 inches). First, the mold 70 is sprayed with mold release solution and allowed to dry. In one variation, approximately 5 grams of Dragon Skin Silicone comprising 2.5 grams of part A and 2.5 grams of part B is mixed. Alternatively, a thermoplastic elastomer such as Kraton CL2003X is used for its cost savings and its ability to be sutured. Approximately 20 microliters of flesh tone color is mixed into the silicone. The core 76 is inserted into the well 80 and the silicone mixture is poured into the mold base 72. The mixture is spread evenly up to a pour line making sure all the wells are filled. The top 74 is placed over the base 72 of the mold 70. Excess silicone mixture is cleaned away and the silicone inside the mold 70 is allowed to dry for approximately one hour under a heat lamp or for two hours without a heat lamp.

After the silicone mixture has dried, the top 74 is removed and the formed skin layer 40 is peeled and removed from the base 72. The core 76 is also removed. The integrally formed umbilical stalk 42 is inverted by passing it through a formed opening 92. Silicone adhesive is provided and delivered using a syringe to the inside of the tube of the umbilical stalk 42. One or more clamps and in one variation, three clamps, such as binder clips, are used to clamp the inverted umbilical stalk 42 closed and sealed to create a bellybutton shape having a star or Y-shaped closure as shown in FIGs. 1 or 2. The bottom-most part of the umbilical stalk 42 is clamped to create a deep umbilicus as opposed to clamping closer to the skin layer bottom surface 64. The skin layer 40 is turned over and excess glue that may have seeped out of the umbilicus 42 is removed. The adhesive is allowed to dry for approximately one hour and the clamps are removed. In one variation, an umbilical shaft 42b is provided. The umbilical shaft 42b is tubular having a central lumen and made of a thin layer of white silicone that is approximately 1 mm thick. The umbilical shaft 42b is glued to the umbilical stalk 42a to extend the umbilicus deeper into the layers and create a more realistic look and feel. The umbilical shaft 42b is glued to the umbilical stalk 42a such that the lumens interconnect. The proximal end of the umbilical shaft 42b is place over the stalk 42a and glued thereto and the distal end of the umbilical shaft 42b is free. In another variation, the distal end of the umbilical shaft is glued or integrally formed with the peritoneum layer 58.

All of the layers are properly oriented in the same direction and aligned such that the apertures 66 and openings 68 are superimposed. Then, with the skin layer 40 inverted and the umbilical stalk 42a either alone or with an extended umbilical shaft 42b is passed through the circular aperture 66 of the fat layer 44 and through the elongate openings 68 of the anterior rectus sheath layer 46, the first rectus muscle layer 48, the second rectus muscle layer 50, and the third rectus muscle layer 52 and then through the circular apertures 66 of the posterior rectus sheath layer 54, the transversalis fascia layer 56 and the peritoneum layer 58 as shown in FIG. 5. In one variation, the umbilicus 42 is left meeting the peritoneum layer 58 or in another variation, the umbilicus 42 is attached with adhesive to the peritoneum layer 58 and yet in another variation, integrally molded with the peritoneum layer 58. The inferior epigastric vein and artery layer 60 is also included. This layer 60 can be formed as layer having a circular aperture 66 with embedded arteries and veins or simply comprise a pair of cylindrical silicone structures, one red and one blue, placed on one side of the midline and another pair of cylindrical silicone structures, one red and one blue in color, placed on the other side of the midline as shown in FIG. 4. The cylindrical silicone structures representing the epigastric veins and arteries are glued to at least one of the adjacent posterior rectus sheath layer 54 and the transversalis fascia layer 56. A price tag holder or other fastener can then be used to connect the layers together as shown in FIG. 5 with the umbilicus 42 shown protruding from the aperture 66 in the bottom-most peritoneum layer 58.

As can be seen in FIG. 5, the skin layer 50 and the peritoneum layer 58 is slightly larger than the other internal layers 44, 46, 48, 50, 52, 54, 56. In particular, the skin layer 50 and peritoneum layer 58 are larger by approximately 32mm (1.25 inches) in length and width. Whereas the internal layers are approximately 165mm (6.5 inches) long and 152mm (6 inches) wide, the peritoneum layer 58 and skin layer 40 is approximately 203mm (8 inches) long and 190mm (7.5 inches) wide. These extra length and width portions are captured between the top and bottom frames of the support 14, pegs in one of the top or bottom frames are passed through apertures in the skin layer 40 formed by mold pegs 90. The peritoneum layer 58 may also include apertures for passing of frame pegs. The top frame and bottom frame are then heat staked together capturing the anatomical portion 12. The resulting model 10 is approximately 38mm (1.5 inches) thick.

The first entry model 10 is then placed inside an opening in the top cover 22 of a laparoscopic trainer 20 and securely attached. Laparoscopic first entry procedures such as the ones discussed in the background of this specification are then practiced on the model 10 employing one or more of the trocar instruments described above creating first entry in any of the locations described above including first entry directly through the umbilicus. Another location for first entry could be within 13mm (0.5 inches) on either side of the midline. Although such first entry is not preferred, the practitioner will advantageously and quickly recognize a mistaken first approach when only the skin layer 42, the fat layer 44 and posterior rectus sheath 54 and peritoneum 58 layers are observed at the linea alba. The absence of a pink-colored first rectus muscle layer 48 should immediately alarm the practitioner during practice that penetration is at a wrong location. Another location for first entry penetration can take place at the left upper quadrant or right upper quadrant. As mentioned above, the left upper quadrant is different from the umbilicus region in that there are muscle layers. While penetrating at the upper right or left quadrants, the practitioner will observe the following layers: the skin layer 40, the fat layer 44, the anterior rectus sheath layer 46, the first rectus muscle layer 48, the second rectus muscle layer 50, the third rectus muscle layer 52, the posterior rectus sheath layer 54, the transversalis fascia layer 56 and the peritoneum layer 58.

The first entry model 10 of the present invention is particularly suited for laparoscopic procedures and may be employed with a laparoscopic trainer 20; however, the invention is not so limited and the first entry model 10 of the present invention can be used alone to practice first entry surgical procedures equally effectively.

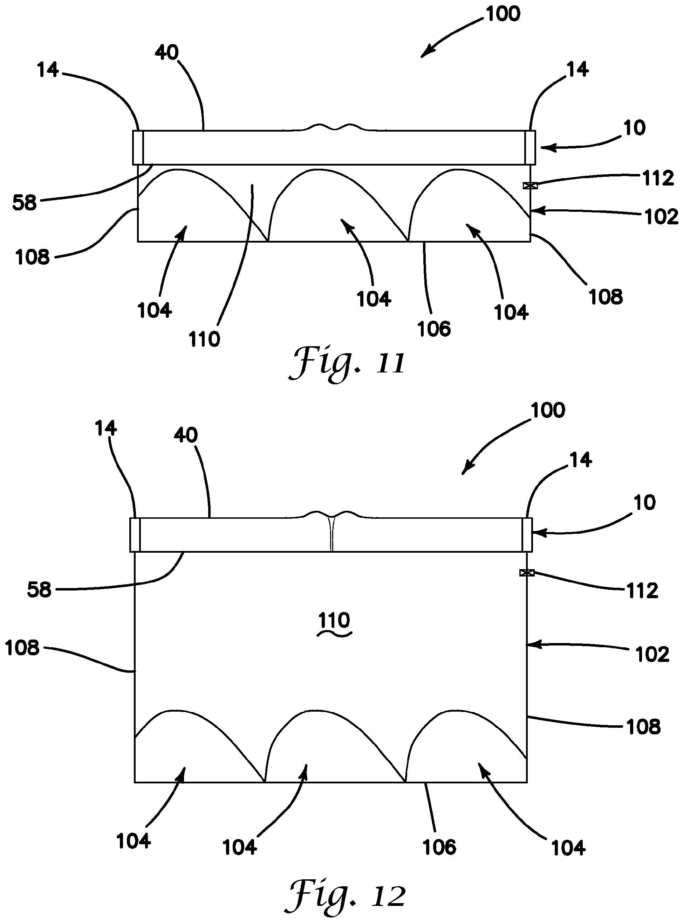

Turning now to FIG. 11, a first entry system 100 will now be described wherein like parts are designated with like reference numerals. The first entry system 100 includes a first entry model 10 of the like described above. The first entry model 10 may include one or more of the layers described above and may or may not include openings 66, 68 and/or umbilicus 42. The first entry model 10 is connected to an organ receptacle 102. The organ receptacle 102 contains one or more real or simulated organs or tissue structures 104. The first entry system 100 may be inserted into a laparoscopic trainer 20 of the like described above. The first entry system 100 is configured to simulate insufflation of the abdominal space to provide a realistic insufflation training experience to the surgical trainee as will be described herein below.

The first entry model 10 includes at least a first simulated tissue layer 40 such as a skin layer 40 at a first end and a second simulated tissue layer 58 such as the peritoneum layer 58 at a second end. Between the first and second simulated tissue layers 40, 58, any number of additional simulated tissue layers and structures may be included as described above. The first entry model 10 includes a lower surface and an upper surface. Typically, the upper surface includes the top surface 62 of the skin layer 40 and the lower surface includes the outer-facing surface of the peritoneum layer 58.

The organ receptacle 102 includes a base 106 interconnected to one or more sidewalls 108 to define an interior 110 with an open top. The organs 104 are disposed inside the interior 110. The receptacle 102 need not have a defined base 106 and defined sidewalls 108. Instead, the base 106 may form an amorphous, bladder-like container with no distinguishable sides with the base 106 defining an interior 110 having an open top or mouth. In such a variation, the open top is sealingly connected to lower surface of the model 10 which typically is the peritoneum layer 58. Alternatively, the open top is connected to or captured between the frame elements of the support 14. In another variation, the receptacle 102 may include a radially outwardly extending flange around the open top. The flange is configured to be captured within the frame elements of the support 14 in order to be connected to the model 10. In another variation, the base 106 is rigid and substantially flat or planar suitable for supporting simulated organs 104 and connected to flexible sidewalls 108. In another variation, the receptacle 102 is at least one layer of elastomeric material having an upper surface and a lower surface defining a thickness. The layer comprises the receptacle 102. The upper surface of the layer is sealingly attached to the lower surface of the first entry model 10. It may be attached with or without adhesive. For example, without adhesive the receptacle 102 layer is capture within the frame support 14 about its perimeter and adjacent to the plurality of layers simulating the abdominal wall. Adhesive may be employed to sealingly attach to the lower surface of the model 10 such that a portion of unadhered or unattached layer is surrounded or encompassed by a portion of the layer that is attached creating an expandable separation or pocket between the model 10 and the layer of the receptacle 102. The wall/layer of the receptacle 102 may be made of transparent material.

The receptacle 102 is sealingly connected to the first entry model 10 such that the interior 110 of the receptacle 102 is sealed against the first entry model 10 leaving a central portion that is unsealed. The central portion or pocket is surrounded by the sealed portion. The receptacle 102 is a pocket. In one variation, the organ receptacle 102 is connected to the first entry model 10 such that the open top is sealed closed against the lowest simulated tissue layer 58. In another variation, the organ receptacle 102 is connected to the support or frame 14 of the first entry model 10. The organ receptacle 102 is connected such that the interior 110 is sealed from the exterior by at least a portion of the first entry model 10 and, in one variation, by the second simulated tissue layer 58 such that the second simulated tissue layer 58 closes or covers at least a portion of the open top of the receptacle 102.

In one variation, the receptacle 102 is completely enclosed and does not have an open top. In such a variation, at least one side surface of the receptacle 102 is adjacent to the first entry model 10 or the at least one side surface of the receptacle 102 itself comprises one of the layers of the first entry model 10 such as the second simulated peritoneum tissue layer 58. In this variation, the receptacle 102 may also include a flange element about its perimeter and configured to be capture within the frame elements of the support 14. In another variation, other fastening means for connecting the receptacle 102 to the model 102 are employed including but not limited to magnets, hook-and-loop type fastener, snaps, flanges, screws, pegs, and friction fit configurations.

The receptacle 102 can be made of any suitable material such as an elastic polymer, elastomer, polymer, silicone, Kraton, latex, rubber, gel, transparent gel, transparent silicone and the like. The receptacle 102 is elastic and can expand when inflated and contract is size when deflated. As such, the receptacle 102 is a balloon-like object. Simulated organs 104 that are placed inside the receptacle 102 can be made of any material such as silicone, Kraton, elastomer, polymer, plastic, rubber, hyrdrogel, mesh material and made include fillings of liquid, water, conductive material, filament and the like. In one variation, the simulated organs 104 include a two dimensional image attached to a three dimensional shape to provide a realistic appearance of the interior of the abdomen. In another variation, the simulated organs 104 comprise only a two dimensional image attached to the inner surface of the receptacle 102 that is smooth. The two dimensional image may be a picture, photograph, drawing of the interior of a patient including organs, tissues and colors. In yet another variation, the simulated organs 104 comprise a two dimensional image attached to the inner surface of the receptacle 102 that is contoured. It is understood that the simulated organs 104 are not limited to the depiction or simulation of organs but may include tissues in general, partial organs and/or colorations that are not readily identifiable as organs or tissue but depict the color of blood, fat, muscle, and/or tumors and the like.

Furthermore, upon sealing the receptacle 102 to the first entry model 10 or prior to attachment of a closed receptacle 102, a negative pressure is created within the interior 110 of the receptacle 102 relative to the exterior. A valve 112 may be provided across the receptacle 102 to create a vacuum inside the receptacle 102. The valve 112 is configured to be connectable to a vacuum source, for example, a mechanical, electro-mechanical and/or hand pump and the like. The receptacle 102 is configured such that with the application of negative pressure, the volume of the interior 110 is reduced as shown in FIG. 11. The reduction in volume of the interior 110 is accomplished by making at least the sidewalls of receptacle 102 from an elastic or flexible plastic material such that the sides of the receptacle 102 are drawn up closer to the first entry model 10, and, in particular, closer to the second simulated tissue layer 58 when a vacuum is applied. Of course, the entire receptacle 102 can be made of an elastic, flexible plastic, or balloon-like material such that the entirety of the receptacle 102 is permitted to be drawn closer to the first entry model 10 in an undeformed condition or upon application of negative pressure. Alternatively, only the sidewalls 108 are retracted under negative pressure with the base 106 being substantially rigid relative to the sidewalls 108. In such a variation, the sidewalls 108 are configured to contract resulting in the base 106 being pulled closer to the first entry model 10 under a vacuum. In any variation, as a result of the application of negative pressure, the simulated organs 104 that are located inside the receptacle 102 will also be drawn closer to the first entry model 10 along with the base 106 as shown in FIG. 11. Hence, the distance between the second simulated tissue layer 58 and the base 106 is reduced.

Since the first entry model 10 is located above the organ receptacle 102, penetration of the first simulated tissue layer 40 by a trocar or other instrument will be followed by penetration of the second simulated tissue layer 58 with continued advancement of the trocar or other instrument. Such penetration will include penetration of any additional intervening layers such as any one or more of the fat layer 44, anterior rectus sheath 46, second rectus muscle layer 48, second rectus muscle layer 50, third rectus muscle layer 52, posterior rectus sheath layer 54, transversalis fascia layer 56, and inferior epigastric vein and artery layer 60 that may be part of the model 10. Upon penetration of the second simulated tissue layer 58 or lowest layer, the vacuum will be broken and the pressure of the interior 110 will equalize with the exterior pressure either through the puncture itself or through an aperture in the distal tip of the trocar or other instrument. The FIOS® trocar manufactured by Applied Medical Resources, Inc. in California advantageously includes a distally located vent hole in the penetrating, transparent tip of the trocar which provides fluid communication between the interior 110 of the receptacle 102 and the exterior or other fluid source. In one variation, the trocar or other instrument includes a stopcock valve at the proximal end of the trocar which the user would open in order to equalize pressure with the interior 110. When the seal of the receptacle 102 is broken by the penetrating trocar or other instrument, or otherwise the pressure is equalized, such as by the penetration of the receptacle 102, the volume of the interior 110 will increase. As the volume of the interior 110 increases, the flexible or elastic sidewalls 102 and/or base 106 will unfurl and the distance between the base 106 and the first entry model 10 will increase. A camera such as a laparoscope disposed inside the trocar or other instrument, will provide to the user a live visualization of the penetration via a video feed connected to a display monitor 34. The penetration of the seal and/or equalization of the pressure will provide a dynamic visual to the user of the organs 104 appearing to drop relative to the first entry model 10 to an insufflated condition of the receptacle 102 shown in FIG. 12. Hence, the present invention provides a simulation of insufflation without the use of insufflation gas.

If the receptacle 102 includes an open top or mouth connected to the model 10 or if the receptacle 102 is an enclosed container, a negative pressure may be generated inside the interior 110 across a valve 112 just prior to demonstration or at the factory before shipment. The user may attach a pump to remove air and create the first configuration. In one variation, the valve 112 is a check valve permitting flow in one direction. In another variation, the valve 112 is a one-way pressure valve that opens to release air from the interior of the receptacle 102 when the receptacle 102 is subjected to sufficient compression pressure to open the valve. When the pressure on the receptacle is released, the valve 112 closes. Hence, prior to use, the user can squeeze the receptacle to release air from the interior of the receptacle 102 across the one-way pressure valve which closes and seals the receptacle 102 after the squeezing on the receptacle 102 is stopped. With the excess air removed from the receptacle 102 the interior volume of the receptacle 102 is reduced from a first volume to a second volume. The sidewall of the receptacle 102 is scrunched around the simulated organs 104 inside the receptacle 102. When the receptacle 102 is punctured, the volume of air in the receptacle returns to the first volume which is larger than the second volume. As the volume of the interior increases, typically under the influence of gravity. The weight of the receptacle 102 and/or simulated organs 104 will be pulled by gravity downwardly away from the model 10. In such a configuration, the receptacle 102 is suspended or hanging from the model 10 with space beneath the receptacle 102 such as inside the laparoscopic trainer 20. The expansion in volume of the interior of the receptacle 102 is a result of stretching of the sidewall of the receptacle 102 or by an unfoldment, unfurling, unwrinkling of the receptacle 102 sidewall in one or more locations. Because the simulated organs 104 are heavier than the receptacle 104, the simulated organs 104 will drop under the influence of gravity from a prior position being drawn up closer to the model 10. The puncture permits air to enter the interior 110 of the receptacle 102 and the receptacle 102 expands downwardly assuming a natural configuration. In essence, air is removed or evacuated from the receptacle 102, for example via a one way valve or other opening, creating a situation wherein the contents of the receptacle 102 are held in place close to the model 10 or lowermost layer of simulated tissue 58 until the user creates an air passageway into the interior 110 of the receptacle 102 at which point the interior opens due to the force of gravity acting on the receptacle and/or simulated organs 104. The air passageway into the interior 110 of the receptacle 102 is created by the insertion of a trocar across the model 10 and into the interior of the receptacle 102 in a simulated medical procedure. The receptacle 102 may include a zipper for accessing the interior 110 for the customized selection and placement of simulated organs 104 inside the receptacle 102 by the user. The simulated organs 104 may be pre-loaded into the receptacle 102 or loaded by the user just prior to use. Also, the pressure differential inside the receptacle 102 may be created by the user on site using a various pumps or, alternatively, the receptacle 102 is sealed and shipped in a ready-to-use state to the user.

In another variation, the negative pressure of the interior 110 relative to the exterior may be restored either through a valve 112 across the receptacle 102 or through the inserted trocar in order to simulate a loss of pneumoperitoneum during the course of a procedure. The restoration of negative pressure may be activated by a teacher while the student is practicing surgical procedures to train the student on how to handle the loss of pressure during a surgical procedure.

The system includes at least one tissue simulation of the like described above including but not limited to two-dimensional constructs such as images or three-dimensional structures that simulate tissue, organs with textures, contours and colors. The tissue simulation is located inside the receptacle pocket may include simulated vasculature, fat, organs, intestines etc. In another variation, the tissue simulation is integrally formed with the receptacle layer. For example, the receptacle layer is formed from a plurality of layers with each layer having the desired size and shape and transparency to simulate tissues and organs encountered in the abdomen of a human being. The tissue simulation may or may not be attached to the receptacle layer/wall. In one variation, the tissue simulation is attached to the lower surface of the receptacle layer. In such a variation, the attached tissue simulation is visible through a transparent receptacle layer. The receptacle layer has a first configuration and a second configuration. While in the first configuration of the receptacle, the tissue simulation inside the receptacle is located proximally to the simulated tissue structure relative to the second configuration wherein while in the second configuration at least part of the tissue simulation inside the receptacle is located distally from simulated tissue structure relative to the first configuration. Fluid is transferable into the receptacle pocket to convert the receptacle from a first configuration to a second configuration. This can be accomplished in several ways. One way is removing air from the pocket creating a vacuum or partial vacuum such that the receptacle pocket layer is withdrawn closer to the penetrable simulated tissue structure. When the penetrable simulated tissue structure is penetrated with a distal tip of a surgical instrument such as the distal tip of an optical obturator, the vacuum is release and pressure is equalized causing the receptacle layer/wall to sag or move away from the penetrable simulated tissue structure especially under weight of the tissue simulations located in the receptacle. The system further includes plugs such as dowel pins sized to fit into the openings created by any previous penetrations so that the system is reusable and subsequent multiple penetrations and demonstrations are possible. Also, one of the layers, preferably one simulating the adipose fat layer, inside the penetrable simulated tissue structure is made of self-sealing foam to help plug the previous penetrations making the structure reusable.

It is understood that various modifications may be made to the above disclosed surgical training device and method without departing from the scope of the invention as claimed in the following claims.

## Claims

1. A surgical training device (100), comprising:
a penetrable simulated tissue structure (12, 40) having an upper surface (62, 40) and a lower surface (58) and comprising at least one layer (40, 44, 46, 48, 50, 52, 54, 56, 58) that simulates a tissue layer;
a receptacle (102) connected to the simulated tissue structure (12, 40); the receptacle (102) having a wall (108, 106) defining an interior (110) of the receptacle (102); and
one or more simulated organs (104) located in the interior (110) of the receptacle (102); the simulated organs (104) being configured to be located proximally to the simulated tissue structure,
**characterized in that** the receptacle (102) is configured to retain a negative pressure inside the interior (110) relative to the exterior of the receptacle (102) and **in that** the surgical training device is configured such that penetration of the one or more of the simulated tissue structure and receptacle (102) releases the negative pressure, resulting in at least part of the one or more simulated organs (104) inside the receptacle (102) to translate distally away from the simulated tissue structure to simulate surgical insufflation of an abdominal cavity.

2. The surgical training device of claim 1 wherein the receptacle (102) includes a top opening; the receptacle (102) being connected to the penetrable simulated tissue structure such that the perimeter of the opening is sealed against the penetrable simulated tissue structure to prevent the transfer of fluid into and out of the interior (110) of the receptacle (102).

3. The surgical training device of claim 1 or 2 wherein the wall (108, 106) of the receptacle (102) completely surrounds the simulated organs (104); the wall (108, 106) defining an exterior of the receptacle (102); and further including a valve (112) located across the wall (108, 106) of the receptacle (102) to provide fluid communication across the exterior and interior (110) of the receptacle (102).

4. The surgical training device of claim 3 wherein the valve (112) is a check-valve or a one-way pressure valve.

5. The surgical training device of any preceding claim further including a support configured to connect the surgical training device to a laparoscopic trainer such that the receptacle (102) is suspended.

6. The surgical training device of any preceding claim wherein the simulated tissue structure includes a plurality of substantially planar layers (40, 44, 46, 48, 50, 52, 54, 56, 58) configured to simulate an abdominal wall.

7. The surgical training device of claim 1 wherein the simulated tissue structure is penetrable with a blunt surgical instrument.

8. A method for simulating surgical insufflation comprising the steps of:
providing a surgical training device as claimed in claim 1;
moving an optical surgical obturator through the simulated tissue structure (12, 40) and into the receptacle (102);
observing the one or more simulated organs (104) inside the receptacle (102) through the distal end of the optical obturator;
breaking the vacuum inside the receptacle (102), causing the one or more simulated organs to move from a position proximal to the simulated tissue structure (12, 40) to a position distal to the simulated tissue structure (12, 40) to simulate insufflation of an abdominal cavity.

9. The method of claim 8 wherein the wall of the receptacle is an elastic wall (108, 106); and
wherein the step of breaking the vacuum inside the receptacle includes the step of expanding the elastic wall (108, 106) of the receptacle (102).

10. The method of claim 8 further including the steps of;
providing a laparoscopic trainer (20) having a cavity (28) and a floor (24) for the cavity (28); and
suspending the training device (100) above the floor (24) of the cavity (28) inside the laparoscopic trainer (20).

## Patentansprüche

1. Eine chirurgische Schulungsvorrichtung (100), die Folgendes beinhaltet:
eine penetrierbare simulierte Gewebestruktur (12, 40) mit einer oberen Oberfläche (62, 40) und einer unteren Oberfläche (58) und die mindestens eine Schicht (40, 44, 46, 48, 50, 52, 54, 56, 58) beinhaltet, die eine Gewebeschicht simuliert;
ein mit der simulierten Gewebestruktur (12, 40) verbundenes Behältnis (102); wobei das Behältnis (102) eine Wand (108, 106) aufweist, die ein Inneres (110) des Behältnisses (102) definiert; und
ein oder mehrere simulierte Organe (104), die sich im Inneren (110) des Behältnisses (102) befinden; wobei die simulierten Organe (104) dazu konfiguriert sind, sich in der Nähe der simulierten Gewebestruktur zu befinden,
**dadurch gekennzeichnet, dass** das Behältnis (102) zum Aufrechterhalten eines Unterdrucks innerhalb des Inneren (110) relativ zu dem Äußeren des Behältnisses (102) ausgelegt ist, und dass die chirurgische Schulungsvorrichtung so ausgelegt ist, dass die Penetration der einen oder mehreren simulierten Gewebestrukturen und des Behältnisses (102) den Unterdruck auflöst, was dazu führt, dass mindestens ein Teil des einen oder der mehreren simulierten Organe (104) innerhalb des Behältnisses (102) distal weg von der simuliertem Gewebestruktur verlagert wird, um die chirurgische Insufflation einer Bauchhöhle zu simulieren.

2. Die chirurgische Schulungsvorrichtung gemäß Anspruch 1, wobei das Behältnis (102) eine obere Öffnung umfasst; wobei das Behältnis (102) so mit der penetrierbaren simulierten Gewebestruktur verbunden ist, dass die äußere Begrenzung der Öffnung gegen die penetrierbare simulierte Gewebestruktur abgedichtet ist, um den Transfer von Fluid in das und aus dem Inneren (110) des Behältnisses (102) zu verhindern.

3. Die chirurgische Schulungsvorrichtung gemäß Anspruch 1 oder 2, wobei die Wand (108, 106) des Behältnisses (102) die simulierten Organe (104) vollkommen umgibt; wobei die Wand (108, 106) ein Äußeres des Behältnisses (102) definiert; und die ferner ein Ventil (112) umfasst, das sich über die Wand (108, 106) des Behältnisses (102) hinweg befindet, um Fluidkommunikation über das Äußere und Innere (110) des Behältnisses (102) hinweg bereitzustellen.

4. Die chirurgische Schulungsvorrichtung gemäß Anspruch 3, wobei das Ventil (112) ein Rückschlagventil oder ein Einweg-Druckventil ist.

5. Die chirurgische Schulungsvorrichtung gemäß einem vorhergehenden Anspruch, die ferner eine Stütze umfasst, die dazu ausgelegt ist, die chirurgische Schulungsvorrichtung mit einer laparoskopischen Schulungseinheit zu verbinden, sodass das Behältnis (102) aufgehängt ist.

6. Die chirurgische Schulungsvorrichtung gemäß einem vorhergehenden Anspruch, wobei die simulierte Gewebestruktur eine Vielzahl von im Wesentlichen planaren Schichten (40, 44, 46, 48, 50, 52, 54, 56, 58) umfasst, die dazu ausgelegt sind, eine Bauchwand zu simulieren.

7. Die chirurgische Schulungsvorrichtung gemäß Anspruch 1, wobei die simulierte Gewebestruktur mit einem stumpfen chirurgischen Instrument penetriert werden kann.

8. Ein Verfahren zum Simulieren einer chirurgischen Insufflation, das die folgenden Schritte beinhaltet:
Bereitstellen einer chirurgischen Schulungsvorrichtung nach Anspruch 1;
Bewegen eines optischen chirurgischen Obturators durch die simulierte Gewebestruktur (12, 40) und in das Behältnis (102) hinein;
Beobachten des einen oder der mehreren simulierten Organe (104) innerhalb des Behältnisses (102) durch das distale Ende des optischen Obturators;
Aufheben des Vakuums in dem Behältnis (102), was dazu führt, dass das eine oder die mehreren simulierten Organe sich von einer Position, die proximal zu der simulierten Gewebestruktur (12, 40) liegt, zu einer Position, die distal zu der simulierten Gewebestruktur (12, 40) liegt, zu bewegen, um die Insufflation einer Bauchhöhle zu simulieren.

9. Das Verfahren gemäß Anspruch 8, wobei die Wand des Behältnisses eine elastische Wand (108, 106) ist; und
wobei der Schritt des Aufhebens des Vakuums innerhalb des Behältnisses den Schritt des Ausdehnens der elastischen Wand (108, 106) des Behältnisses (102) umfasst.

10. Das Verfahren gemäß Anspruch 8, das ferner die folgenden Schritte umfasst:
Bereitstellen einer laparoskopischen Schulungseinheit (20) mit einer Höhle (28) und einem Boden (24) für die Höhle (28); und
Aufhängen der Schulungsvorrichtung (100) über dem Boden (24) der Höhle (28) innerhalb der laparoskopischen Schulungseinheit (20).

## Revendications

1. Dispositif de formation chirurgicale (100), comprenant :
une structure de tissu simulée pénétrable (12, 40) ayant une surface supérieure (62, 40) et une surface inférieure (58) et comprenant au moins une couche (40, 44, 46, 48, 50, 52, 54, 56, 58) qui simule une couche de tissu ;
un réceptacle (102) rattaché à la structure de tissu simulée (12, 40), ce réceptacle (102) ayant une paroi (108, 106) définissant un intérieur (110) du réceptacle (102) ; et
un ou plusieurs organes simulés (104) situés dans l'intérieur (110) du réceptacle (102), ces organes simulés (104) étant configurés de façon à être situés de manière proximale par rapport à la structure de tissu simulée,
**caractérisé en ce que** le réceptacle (102) est configuré de façon à maintenir une pression négative dans l'intérieur (110) par rapport à l'extérieur du réceptacle (102) et **en ce que** le dispositif de formation chirurgicale est configuré de manière à ce que la pénétration d'au moins soit la structure de tissu simulée, soit le réceptacle (102) relâche la pression négative, ayant pour effet qu'au moins une partie du ou des organes simulés (104) à l'intérieur du réceptacle (102) se translate de manière distale par rapport à la structure de tissu simulée afin de simuler une insufflation chirurgicale d'une cavité abdominale.

2. Dispositif de formation chirurgicale selon la revendication 1, dans lequel le réceptacle (102) comprend une ouverture supérieure, le réceptacle (102) étant rattaché à la structure de tissu simulée pénétrable de manière à ce que le périmètre de l'ouverture soit scellé contre la structure de tissu simulée pénétrable afin d'empêcher le transfert de fluide dans et hors de l'intérieur (110) du réceptacle (102).

3. Dispositif de formation chirurgicale selon la revendication 1 ou 2, dans lequel la paroi (108, 106) du réceptacle (102) entoure complètement les organes simulés (104), la paroi (108, 106) définissant un extérieur du réceptacle (102) ; et comprenant en outre une soupape (112) située à travers la paroi (108, 106) du réceptacle (102) afin de fournir une communication fluidique entre l'extérieur et l'intérieur (110) du réceptacle (102).

4. Dispositif de formation chirurgicale selon la revendication 3, dans lequel la soupape (112) est une soupape anti-retour ou une soupape de sécurité unidirectionnelle.

5. Dispositif de formation chirurgicale selon l'une quelconque des revendications précédentes, comprenant en outre un support configuré de façon à rattacher le dispositif de formation chirurgicale à un simulateur laparoscopique de manière à ce que le réceptacle (102) soit suspendu.

6. Dispositif de formation chirurgicale selon l'une quelconque des revendications précédentes, dans lequel la structure de tissu simulée comprend une pluralité de couches essentiellement planaires (40, 44, 46, 48, 50, 52, 54, 56, 58) configurées de façon à simuler une paroi abdominale.

7. Dispositif de formation chirurgicale selon la revendication 1, dans lequel la structure de tissu simulée est pénétrable avec un instrument chirurgical émoussé.

8. Procédé pour simuler une insufflation chirurgicale comprenant les étapes consistant à :
prévoir un dispositif de formation chirurgicale selon la revendication 1 ;
bouger un obturateur chirurgical optique à travers la structure de tissu simulée (12, 40) et dans le réceptacle (102) ;
observer le ou les organes simulés (104) à l'intérieur du réceptacle (102) à travers l'extrémité distale de l'obturateur optique ;
briser le vide à l'intérieur du réceptacle (102), faisant en sorte que le ou les organes simulés bougent d'une position proximale par rapport à la structure de tissu simulée (12, 40) dans une position distale par rapport à la structure de tissu simulée (12, 40) afin de simuler l'insufflation d'une cavité abdominale.

9. Procédé selon la revendication 8, dans lequel la paroi du réceptacle est une paroi élastique (108, 106) ; et
dans lequel l'étape consistant à briser le vide à l'intérieur du réceptacle comprend l'étape consistant à dilater la paroi élastique (108, 106) du réceptacle (102).

10. Procédé selon la revendication 8, comprenant en outre les étapes consistant à :
fournir un simulateur laparoscopique (20) ayant une cavité (28) et un fond (24) pour la cavité (28) ; et à
suspendre le dispositif de formation (100) au-dessus du fond (24) de la cavité (28) à l'intérieur du simulateur laparoscopique (20).
